# EUROPÄISCHE PATENTSCHRIFT

(11) **EP 2 934 361 B1**
(45) Veröffentlichungstag und Bekanntmachung des Hinweises auf die Patenterteilung: **26.09.2018**
(21) Anmeldenummer: 13828769.3
(22) Anmeldetag: 12.12.2013
(51) Int. Cl.: A61B 34/30, B25J 18/00

(54) **HALTERUNGS- UND POSITIONIERVORRICHTUNG EINES CHIRURGISCHEN INSTRUMENTS FÜR DIE MINIMAL-INVASIVE CHIRURGIE SOWIE EIN CHIRURGISCHES ROBOTERSYSTEM**
RETAINING AND POSITIONING DEVICE OF A SURGICAL INSTRUMENT FOR MINIMALLY INVASIVE SURGERY AND SURGICAL ROBOT SYSTEM
DISPOSITIF DE FIXATION ET DE POSITIONNEMENT D'UN INSTRUMENT CHIRURGICAL POUR LA CHIRURGIE MINI-INVASIVE, AINSI QUE SYSTÈME DE ROBOT CHIRURGICAL

(30) Priorität: 20.12.2012 DE 102012025099; 14.03.2013 DE 102013004459
(43) Veröffentlichungstag der Anmeldung: 28.10.2015
(73) Patentinhaber: avateramedical GmbH, 07745 Jena (DE)
(72) Erfinder: SEEBER, Marcel, 07745 Jena (DE); KARGUTH, Andreas, 99869 Tüttleben (DE); TROMMER, Christian, 99310 Wipfratal (DE)
(74) Vertreter: Patentanwälte Geyer, Fehners & Partner mbB
(86) Internationale Anmeldenummer: PCT/DE2013/000803
(87) Internationale Veröffentlichungsnummer: WO 2014/094716

(56) Entgegenhaltungen:
- EP-A2- 2 332 484
- WO-A2-2012/044869
- US-A1- 2007 137 371
- US-A1- 2011 071 473

## Beschreibung

Die vorliegende Erfindung betrifft eine Halterungs- und Positionierungsvorrichtung eines chirurgischen Instruments und ein chirurgisches Robotersystem bzw. Telemanipulator für die minimal-invasive Chirurgie und insbesondere der Laparoskopie.

Robotersysteme oder auch Telemanipulatoren für die minimal-invasive Chirurgie, insbesondere für die laparoskopische Chirurgie, ersetzen die vom Chirurgen üblicherweise manuell geführten Operations-Instrumente, wie beispielsweise chirurgische Instrumente, Endoskop bzw. Kamera, durch eine motorische Positionierung. Die einzusetzenden Operations-Instrumente werden über einen bzw. mehrere Trokare in das Innere eines Patienten geführt. Als Trokar wird ein Instrument bezeichnet, mit dessen Hilfe der Chirurg in der minimal-invasiven Chirurgie ein Zugang zu der Körperhöhle des Patienten (üblicherweise des Bauchraumes oder des Brustraumes) schafft, wobei der Zugang durch ein Rohr, einen sog. Tubus, offengehalten wird. Die in dem Robotersystem vorgesehene Halterung einer Bewegungsmechanik und Steuerungslogik ermöglicht die Bewegung der Operations-Instrumente um einen Pivotalpunkt herum in 2 Freiheitsgraden (x, y) sowie eine translatorische Bewegung der Operations-Instrumente entlang der Instrumentenachse (z). Als Pivotalpunkt wird der invariante Punkt der Bewegung in 2 Freiheitsgraden (x, y) bezeichnet. Dieser Pivotalpunkt liegt idealerweise im oder in der Nähe des Durchstoßpunktes des Trokars durch die Bauchdecke des Patienten. Die Steuerungslogik eines Robotersystems muss den Pivotalpunkt kennen bzw. der Pivotalpunkt muss durch die konstruktive Ausführung der Bewegungsmechanik definiert sein, um eine Bewegung des Operations-Instrumentes so zu begrenzen, dass die biomechanische Belastung des Gewebes um den Trokar herum möglichst gering ist.

Aus dem Stand der Technik bekannte Robotersysteme basieren auf Roboterarmen mit einer aktiven Bewegung eines Operations-Instrumentes, welche einerseits einen großen Bauraum benötigen und wobei aufgrund der typischen Ausführungsformen durch die Bewegungsabläufe der Roboterarme Kollisionen kaum zu vermeiden sind.

Die Dokumente US 2007/137371 A1 und WO 2012/044869 A2 offenbaren beispielhafte Robotersysteme.

Während eines minimal-invasiven chirurgischen Eingriffs werden mindestens zwei, in der Regel drei bis vier chirurgische Instrumente, wie Greifer, Schere, Nadelhalter, Sezierer, sowie eine Kamera bzw. ein Endoskop verwendet, die jeweils über einen gesonderten Trokar in das Körperinnere des Patienten geführt werden. Das bedeutet, dass für jedes eingesetzte Operations-Instrument ein Roboterarm vorhanden ist, der die Positionen der Roboterarme und die aktive Bewegung des Instruments steuert.

Nachteil der Lösungen aus dem Stand der Technik ist, dass aufgrund der raumeinnehmenden Konstruktion die Positionierbarkeit der Instrumente eingeschränkt ist und der Zugang zum Patienten für das OP-Personal, z.B. den assistierenden Arzt und die OP-Schwester, nur eingeschränkt möglich ist.

Ein weiterer Nachteil ist, dass der invariante Punkt bei bekannten Systemen durch eine mechanische Kopplung zwischen Trokar und Roboterarm immer zwingend gegeben ist.

Aufgabe der vorliegenden Erfindung ist es daher, einen Manipulatorarm zur Positionierung eines chirurgischen Instruments und ein chirurgisches Robotersystem bereitzustellen, die bzw. das eine hohe Variabilität vorsieht und nur einen geringen Bauraum benötigt bzw. kleiner und leichter in seiner Ausführung ist und dabei wahlweise die mechanische Kopplung des Trokars mit dem Manipulatorarm ermöglicht bzw. auch ohne diese mechanische Kopplung des Trokars mit dem Manipulatorarm auskommt.

Eine weitere Aufgabe der vorliegenden Erfindung ist es, ein Robotersystem bereitzustellen, das eine größeren Einstellbereich der Vorpositionierung für eine Halterungsvorrichtung eines Manipulatorarmes bietet. Bei Verwendung von zwei oder mehr Halterungsvorrichtungen für Manipulatorarme sind damit flexiblere Positionierungsmöglichkeiten relativ zueinander möglich.

Diese Aufgaben werden durch die vorliegende Erfindung gemäß den Merkmalen von Anspruch 1 durch eine Halterungs- und Positioniervorrichtung eines chirurgischen Instruments für die minimal-invasive Chirurge, insbesondere zur Verwendung innerhalb eines chirurgischen Robotersystems, gelöst, welche
eine erste Drehachse umfasst, um welche ein Halteelement drehbar angeordnet ist, wobei sich die erste Drehachse mit der Längsachse zumindest eines chirurgischen Instruments in einem Pivotalpunkt stets dadurch schneidet, dass an dem Halteelement ein Schubantrieb angebracht ist, welcher eine Instrumentenantriebseinheit um den Pivotalpunkt drehbar anordnet, und wobei eine Teleskopeinrichtung an der Instrumentenantriebseinheit vorgesehen ist, durch welche das chirurgische Instrument und/oder das Endoskop entlang dessen Längsachse mittels einer Führungseinrichtung in den Körper derart translatorisch bewegbar ist, dass die Längsachse des chirurgischen Instruments gegenüber der Teleskopeinrichtung variabel einstellbar ist.

Ferner werden die Aufgaben durch die vorliegende Erfindung gemäß den Merkmalen von Anspruch 11 durch ein chirurgisches Robotersystem mit mehreren Roboterarmen gelöst, an welchen zumindest ein chirurgisches Instrument und/oder ein Endoskop für die minimal-invasive Chirurgie anordenbar ist, zumindest zwei Halterungs- und Positioniervorrichtungen an einem im wesentlichen quer zu den Halterungs- und Positioniervorrichtungen verlaufenden Halterungsträgersystem angebracht sind, wobei das Halterungsträgersystem aus jeweils einer Koppelstelle für jede Halterungs- und Positioniervorrichtung aufgebaut ist, und wobei die Koppelstellen jeweils fest oder über Gelenke miteinander verbunden sind.

Weitere vorteilhafte Ausgestaltungen der Erfindung sowie des erfindungsgemäßen chirurgischen Robotersystems ergeben sich aus den Unteransprüchen analog zum Manipulatorarm zur aktiven Positionierung für ein chirurgisches Instrument. Dies ergibt sich insbesondere dadurch, dass der erfindungsgemäße Manipulatorarm zur aktiven Positionierung eines chirurgischen Instrumentes mit einem Robotersystem kombiniert bzw. nachgerüstet werden kann. Erfindungsgemäß können die Begriffe Robotersystem und Telemanipulator synonym verwendet werden.

Es ist von Vorteil, dass die erfindungsgemäße Instrumentenantriebseinheit an der Teleskopeinrichtung mittels eines Instrumentendrehpunkts derart drehbar gelagert ist, dass die Teleskoplängsachse der Teleskopeinrichtung gegenüber der Längsachse des chirurgischen Instruments in Abhängigkeit des Schubantriebs (5) variabel ist.

Eine weitere Ausführungsform der Erfindung ist derart ausgebildet, dass die Teleskopeinrichtung mehrere Teleskopelemente aufweist, wobei der Instrumentendrehpunkt an dem Teleskopelement angeordnet ist, welches den größten Verstellbereich aufweist.

Gemäß einer bevorzugten Ausführungsform weist die Führungseinrichtung zumindest eine Instrumentenführung auf, in welcher sich der Schaft des chirurgischen Instruments hindurch erstreckt.

Ein besonderer Vorteil besteht darin, dass der Schubantrieb derart an der Teleskopeinrichtung mittels eines Schubantriebsaufnahmepunkt angebracht ist, dass sich die Drehbewegung der Instrumententrägereinheit um den Pivotalpunkt dadurch ergibt, dass eine Kopplungseinrichtung einen Kopplungsdrehpunkt aufweist, welcher mit dem Haltelement fest verbunden ist. Die Drehung der Instrumententrägereinheit mit den Instrumenten sowohl um den Pivotalpunkt als auch um den Kopplungsdrehpunkt ermöglicht es, dass das Halteelement gegenüber dem Pivotalpunkt im wesentlichen konstant angeordnet ist.

Gemäß einer bevorzugten Ausführungsform ist die Halterungs- und Positioniervorrichtung derart ausgelegt, dass die Instrumentenantriebseinheit das chirurgische Instrument in mehreren Freiheitsgraden bewegt, wobei die Ansteuerung der Instrumentenantriebseinheit mittels Steuer- und Versorgungsleitungen, welche durch das Haltelement und den Schubantrieb hindurch geführt werden, über eine Steuereinheit durch den Operateur erfolgt.

Die erste Drehachse wird insbesondere dadurch gebildet, dass eine Antriebseinheit vorgesehen ist, welche das chirurgische Instrument steuert, wobei die Antriebseinheit an einem Roboterarm anbringbar ist, und wobei ein Drehgelenk zwischen der Antriebseinheit und dem Halteelement vorgesehen ist.

Eine weitere Ausführungsform ist derart ausgebildet, dass ein Koppelelement am Halteelement angebracht ist, welches am distalen Ende am Pivotalpunkt mit einer Instrumentenführung drehbar verbunden ist. Dadurch wird der Pivotalpunkt gegenüber dem Halteelement zusätzlich mechanisch vorgegeben, sodass eine zusätzliche Fixierung des Pivotalpunkts ermöglicht wird.

Des Weiteren kann die vorliegende Erfindung dadurch erweitert werden, dass mehrere chirurgische Instrumente durch einen einzigen Trokar hindurch in das Körperinnere geführt werden, wobei für jedes chirurgische Instrument eine separate Instrumentenantriebseinheit vorgesehen ist, und wobei insbesondere die chirurgischen Instrumente in Längsrichtung bogenförmig ausgebildet ist.

Wenn das Halteelement mittels einer Vorpositioniereinrichtung in seiner Ausgangslage anpassbar ist, wobei die Vorpositioniereinrichtung ein oder mehrere Vorpositionierelemente aufweist, welche jeweils über zumindest eine Drehachse in ihrer Position voreinstellbar sind, wobei insbesondere vier Vorpositionierelemente mit zueinander in Reihe variablen Positionen voreinstellbar sind, so kann die Halterungs- und Positioniervorrichtung in einer gewünschten Position voreingestellt werden.

Das chirurgische Robotersystem gemäß der Erfindung kann ferner dadurch weitergebildet werden, dass das Halterungsträgersystem mittels einer Koppelträgerverbindung mit einer im wesentlichen vertikal verlaufenden Hauptträgereinrichtung zur Abstützung gegenüber einem Festlager verbunden ist, welches beweglich angeordnet sein kann oder gegenüber einem feststehenden oder verfahrbaren Operationstisch vorgegeben ist.

Gemäß einer weiteren Ausbildungsform der Erfindung weist das chirurgische Robotersystem eine zentrale Steuereinheit auf, welche mit jeder der Halterungs- und Positioniervorrichtungen mit den entsprechenden chirurgischen und/oder Endoskopen in Verbindung steht und mit einer Bedieneinheit zur Eingabe von Befehlen in Form von Steuerdaten eines Operateurs gekoppelt ist, welche mittels einer Visualisierungseinheit Bilddaten von einem oder mehrerer Endoskopen darstellt.

Darüber hinaus ist es von Vorteil, dass die Steuereinheit und die Bedieneinheit mit einem verfahrbaren Operationstisch gekoppelt sind, wobei sowohl die Bilddaten als auch die Steuerdaten in Abhängigkeit der vorgegebenen Positionen der Halterungs- und Positioniervorrichtung sowie des Operationstisches verarbeitet werden.

Die Erfindung wird durch den anhängenden unabhängigen Anspruch 1 beschränkt. Weitere bevorzugte Ausführungsformen können den abhängigen Ansprüchen entnommen werden.

Die vorliegende Erfindung wird rein beispielhaft durch die beigefügten Figuren realisiert. Es zeigt:
Figur 1a eine schematische Ansicht des erfindungsgemäßen Manipulatorarmes zur aktiven Positionierung eines chirurgischen Instruments, welches über eine schwenkbar gelagerte Antriebseinheit mit einem Teleskopausleger verbunden ist, inklusive des Koppelelementes zwischen der Führungseinrichtung zur Durchführung eines chirurgischen Instruments und der konstruktiven Vorrichtung zur Realisierung der zweiten Drehachse;
Figur 1b eine schematische Ansicht des erfindungsgemäßen Manipulatorarmes zur aktiven Positionierung eines chirurgischen Instruments, welches über eine schwenkbar gelagerte Antriebseinheit mit einem Teleskopausleger verbunden ist, inklusive des Koppelelementes zwischen der Führungseinrichtung zur Durchführung eines chirurgischen Instruments und der konstruktiven Vorrichtung zur Realisierung der zweiten Drehachse;
Figur 2a eine weitere schematische Ansicht des erfindungsgemäßen Manipulatorarmes zur aktiven Positionierung eines chirurgischen Instruments, welches über eine schwenkbar gelagerte Antriebseinheit mit einem Teleskopausleger verbunden ist, inklusive des Koppelelementes zwischen der Führungseinrichtung zur Durchführung eines chirurgischen Instruments und der konstruktiven Vorrichtung zur Realisierung der zweiten Drehachse, aus welcher die Schubbewegung zur Erzeugung der Drehbewegung mittels Koppelgelenk um die zweite Drehachse ersichtlich wird;
Figur 2b eine weitere schematische Ansicht des erfindungsgemäßen Manipulatorarmes zur aktiven Positionierung eines chirurgischen Instruments, welches über eine schwenkbar gelagerte Antriebseinheit mit einem Teleskopausleger verbunden ist, inklusive des Koppelelementes zwischen der Führungseinrichtung zur Durchführung eines chirurgischen Instruments und der konstruktiven Vorrichtung zur Realisierung der zweiten Drehachse, aus welcher die Schubbewegung zur Erzeugung der Drehbewegung mittels Koppelgelenk um die zweite Drehachse ersichtlich wird;
Figur 3a eine schematische Ansicht des erfindungsgemäßen Manipulatorarmes zur aktiven Positionierung eines chirurgischen Instruments, welches über eine schwenkbar gelagerte Antriebseinheit mit einem Teleskopausleger verbunden ist, ohne das Koppelelement gemäß Figur 1a;
Figur 3b eine schematische Ansicht des erfindungsgemäßen Manipulatorarmes zur aktiven Positionierung eines chirurgischen Instruments, welches über eine schwenkbar gelagerte Antriebseinheit mit einem Teleskopausleger verbunden ist, ohne das Koppelelement gemäß Figur 13;
Figur 4 eine schematische Ansicht des erfindungsgemäßen Manipulatorarmes zur aktiven Positionierung eines chirurgischen Instruments, ohne das Koppelelement gemäß Figur 1, aus welcher die Schubbewegung zur Erzeugung der Drehbewegung mittels Koppelgelenk um die zweite Drehachse sowie die Ankopplung der Instrumentenantriebseinheit ersichtlich wird;
Figur 5a eine Draufsicht auf den erfindungsgemäßen Manipulatorarmes zur aktiven Positionierung eines chirurgischen Instruments in der Ausführungsform Teleskoparm rechts;
Figur 5b eine Draufsicht auf den erfindungsgemäßen Manipulatorarmes zur aktiven Positionierung eines chirurgischen Instruments in der Ausführungsform Teleskoparm links;
Figur 6 eine schematische Ansicht des erfindungsgemäßen Manipulatorarmes zur aktiven Positionierung eines chirurgischen Instruments in den Ausführungsformen Teleskoparm rechts und Teleskoparm links zur gemeinsamen Verwendung mit einem Single-Port-Trokar;
Figur 7 eine schematische Ansicht der erfindungsgemäßen, flexibel einstellbaren Trägerstruktur;
Figur 8 eine schematische Ansicht der erfindungsgemäßen Vorpositionierungseinrichtung;
Figur 9 eine schematische Ansicht der erfindungsgemäßen flexibel einstellbaren Trägerstruktur mit einer angeschlossenen erfindungsgemäßen Vorpositionierungseinrichtung an welcher ein erfindungsgemäßer Manipulatorarm zur aktiven Positionierung eines chirurgischen Instrumentes befestigt ist;
Figur 10 eine schematische Seitenansicht eines übergeordneten Tragesystems an welcher das erfindungsgemäße flexibel einstellbare Trägersystem mit insgesamt vier angeschlossenen erfindungsgemäßen Vorpositionierungseinrichtung, an welcher jeweils ein erfindungsgemäßer Manipulatorarm zur aktiven Positionierung eines chirurgischen Instrumentes befestigt ist;
Figur 11 eine schematische Frontansicht des übergeordneten Tragesystems an welcher das erfindungsgemäße flexibel einstellbare Trägersystem mit insgesamt vier angeschlossenen erfindungsgemäßen Vorpositionierungseinrichtung, an welcher jeweils ein erfindungsgemäßer Manipulatorarm zur aktiven Positionierung eines chirurgischen Instrumentes befestigt ist;
Figur 12 eine schematische Gesamtansicht der Verwendung des übergeordneten Tragesystems in einem chirurgischen Robotersystem für den Einsatz in der minimal-invasiven Chirurgie, wie z.B. der Laparoskopie.
Figur 13a eine schematische Ansicht des Manipulatorarmes, an welchem eine erfindungsgemäße Instrumenten-Führungseinrichtung am Teleskopausleger angebracht ist.
Figur 13b eine weitere schematische Ansicht des Manipulatorarmes von Figur 13 a in einer anderen Winkelstellung, an welchem eine erfindungsgemäße Instrumenten-Führungseinrichtung am Teleskopausleger angebracht ist.
Figuren 14a, 14b zeigen schematisch als Ausschnitt die Ausführungsform von Figur 13 a und 13 b hinsichtlich der Ankopplung der Führungseinrichtung 90.
Figuren 15a, 15b zeigen eine gegenüber Figur 14a und 14b verschobene Position des chirurgischen Instrumentes 9.

Die vorliegende Erfindung wird nachfolgend beispielhaft im Detail unter Bezugnahme auf die Figuren beschrieben:

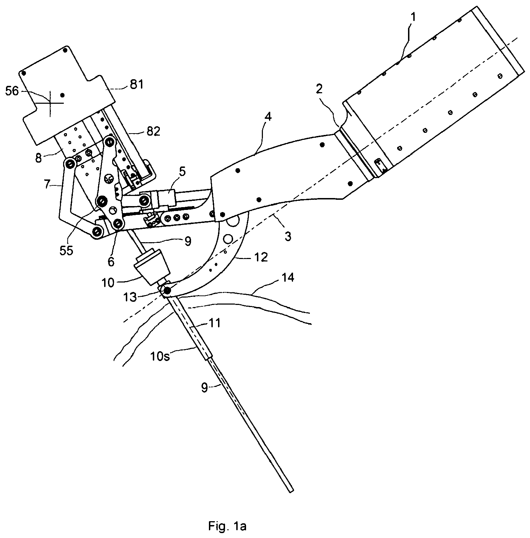

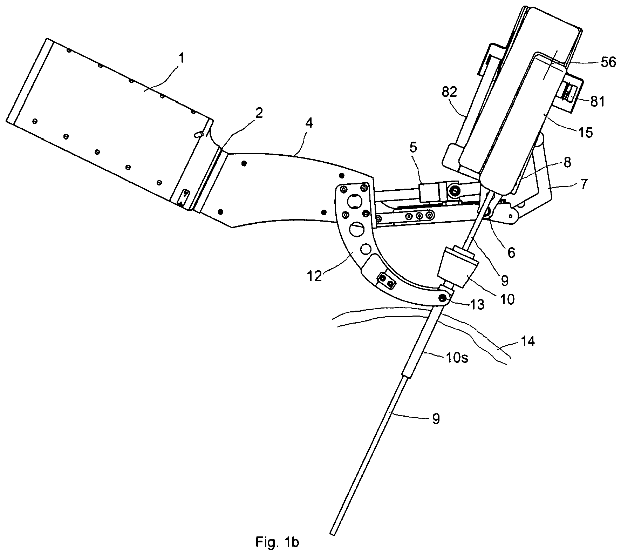

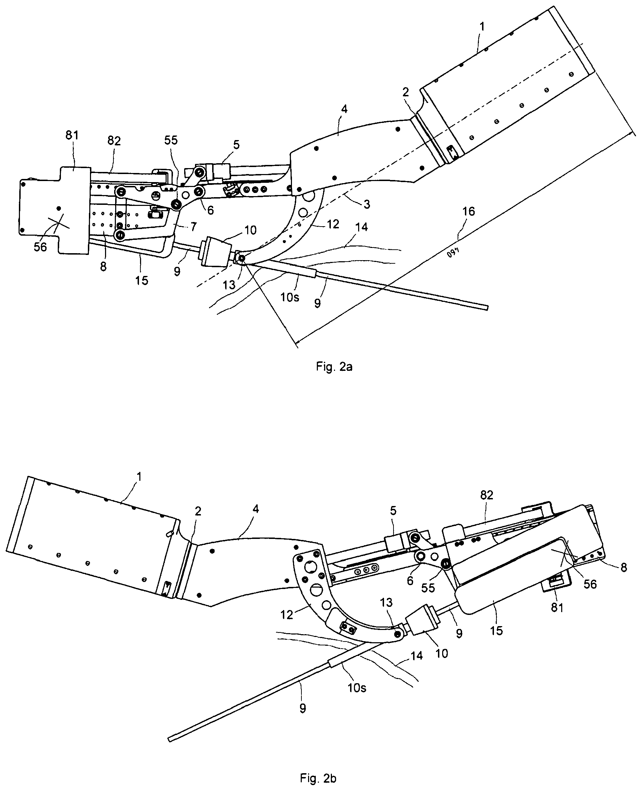

Figur 1a, Figur 2a, Figur 1b und Figur 2b zeigen einen erfindungsgemäßen Manipulatorarm zur aktiven Positionierung eines chirurgischen Instruments 9 inklusive des Koppelelementes 12 zwischen der Führungseinrichtung 10 zur Durchführung eines chirurgischen Instruments 9 und der konstruktiven Vorrichtung 4 zur Realisierung der zweiten Drehachse. Während eines minimal-invasiven, laparoskopischen Eingriffs kommen in der Regel 4 Operations-Instrumente zum Einsatz, hiervon 3 chirurgische Instrumente und 1 Kamera bzw. Endoskop, die über das Telemanipulatorsystem vom Operateur gesteuert werden. Erfindungsgemäß sind demnach vorzugsweise 4 Ausführungen eines Manipulatorarmes im System vorhanden. Es versteht sich jedoch, dass auch Ausführungsformen mit 1 bis 3 oder mehr als 4 Manipulatorarmen gemäß der vorliegenden Erfindung vorgesehen sein können, wobei jeder Manipulatorarm zumindest eine Halterungs- und Positioniervorrichtung gemäß der Erfindung aufweist. Jeder Manipulatorarm weist den Freiheitsgrad 3 auf zur Realisierung von Schwenkbewegungen eines über eine Instrumentenantriebseinheit 15 angekoppelten Instrumentes 9 in x- und y-Richtung sowie für eine translatorische Bewegung in z-Richtung. Dazu besteht jeder Manipulatorarm aus einer ersten Antriebseinheit 1, die über das Drehgelenk 2 eine Drehbewegung von mindestens ±120° um die Drehachse 3 ausgehend von der Nullpunktlage ermöglicht. Diese Drehbewegung um die Drehachse 3 führt zu einer Verkippung der angekoppelten konstruktiven Vorrichtung bestehend aus den Elementen 4, 5, 6, 7, 8, 12 um einen invarianten Punkt 13, dem sogenannten Pivotalpunkt. Das Haltelement 4 trägt einen Schubantrieb 5, welcher eine zweite Drehbewegung um einen zweiten Drehpunkt 6, orthogonal zur Drehachse 3 realisiert. Das Koppelelement 12 zwischen dem Halteelement 4 und der Durchführung 10 für ein chirurgisches Instrument 9 ist am Pivotalpunkt 13 so mit der Durchführung 10 verbunden, dass die Drehachse 3 durch diesen Pivotalpunkt 13 geht und die Durchführung 10 zwangsgeführt um die Drehachse 3 die Verkippung ausführt. Die Durchführung 10 realisiert den Zugang durch die Bauchdecke 14 eines Patienten für ein chirurgisches Instrument 9. Über einen Schubantrieb 5 erfolgt eine Krafteinleitung auf eine Koppelführung 7 im Drehpunkt 55, welche eine Rotation der Koppelführung 7 um den Drehpunkt 6 um mindestens ±60° realisiert. Insbesondere dient die Durchführung 10 als Führungseinrichtung für das chirurgische Instrument 9 und weist einen Führungschsft 10s auf, der als Instrumentenführung des Instruments 9 dient und bevorzugt einstückig mit der Durchführung 10 ausgebildet ist.

An der Koppelführung 7 ist ein Teleskopausleger 8 angeordnet. Der Teleskopausleger 8 weist einen Stellantrieb 81 auf. Die Versorgungs- und Steuerleitungen für den Stellantrieb 81 des Teleskopauslegers 8 werden entlang des Schubantriebes 5 durch das Haltelement 4 und die Antriebseinheit 1 hindurch geführt. Die Versorgungs- und Steuerleitungen für den Schubantrieb 5 werden durch das Haltelement 4 und die Antriebseinheit 1 hindurch geführt.

Am Teleskopausleger 8 ist einen Instrumentenantriebseinheit 15 drehbar angeordnet, wie aus Fig. 2a bzw. 2b ersichtlich ist. Die Instrumentenantriebseinheit 15 dient zur Realisierung des Freiheitsgrades 4 eines daran angekoppelten Instrumentes 9. Dazu ist eine Instrumentenantriebseinheit 15 mit entsprechenden Stellantrieben ausgestattet. Die Versorgungs- und Steuerleitungen für die Stellantriebe der Instrumentenantriebseinheit 15 werden über den Teleskopausleger 8, entlang des Schubantriebes 5 durch das Haltelement 4 und die Antriebseinheit 1 hindurch geführt.

Eine Verkippung des Koppelelementes 7 führt zu einer Kippbewegung des daran befestigten Teleskopauslegers 8 um die Drehachse 6 und damit zu einer Verkippung der Instrumentenantriebseinheit 15 und des daran gekoppelten chirurgischen Instrumentes 9. Dies führt zu einer Kippbewegung der Durchführung 10 in einer orthogonalen Achse zur Drehachse 3 um den Pivotalpunkt 13 (siehe Figur 2a). Die resultierende Lage der Instrumentenlängsachse 11 entspricht der Achse zwischen einem Instrumentendrehpunkt 56 der Instrumentenantriebseinheit 15 am Teleskopausleger 8 und dem Pivotalpunkt 13. Das chirurgische Instrument 9 wird mittels der Durchführung 10 entlang der Instrumentenlängsachse 11 derart zwangsgeführt, das mittels der Antriebe 1 und 5 eine pivotale Kippbewegung des chirurgischen Instrumentes 9 um den Pivotalpunkt 13 in orthogonal zueinander liegenden Achsen realisiert wird. An der Koppelführung 7 ist ein Teleskopausleger 8 derart angeordnet, dass das am Teleskopausleger 8 mittels der Instrumentenantriebseinheit 15 befestigte chirurgische Instrument 9 entlang der Instrumentenlängsachse 11 durch die Durchführung 10 und damit gegenüber der Bauchdeck 14 verschoben werden kann. Die gesamte konstruktive Ausführung kann äußerst kompakt realisiert werden. Chirurgische Instrumente 9 weisen typischerweise einen Durchmesser von 5 bis 10mm und eine Länge von 250 bis 300mm auf. Die erfindungsgemäße Ausführungsform des Teleskopauslegers 8 ist so gestaltet, das ein chirurgisches Instrument 9 um vorzugsweise mindestens 250mm entlang seiner Instrumentenlängsachse 11 gegenüber der Durchführung 10 verschoben werden kann und das im Falle der maximalen Eintauchtiefe des chirurgischen Instrumentes 9 in die Durchführung 10 der Teleskopausleger 8 seine minimalste Länge aufweist, d.h. nur unwesentlich über das proximale Ende des chirurgischen Instrumentes 9 hinausragt, und damit die Kollisionsgefahr zwischen verschiedenen chirurgischen Instrumenten 9 bzw. Teleskopauslegern 8 von nebeneinander angeordneten Manipulatorarmen aufgrund der auszuführenden Pivotalbewegungen minimiert wird. Die gesamte konstruktive Ausführung weist im Vergleich zum Stand der Technik einen deutlich minimierten Bauraumbedarf aus. Die komplette Baulänge 16 eines erfindungsgemäßen Manipulatorarmes gemessen von der Antriebseinheit 1 bis zum Pivotalpunkt 13 beträgt vorzugsweise weniger als 500mm. Die Ausführung mit dem Koppelelement 12 zur Zwangsführung des Pivotalpunktes 13 an der Durchführung 10 ermöglicht den Einsatz des erfindungsgemäßen Manipulatorarmes auch bei offenen, nicht-minimal-invasiv ausgeführten Operationen.

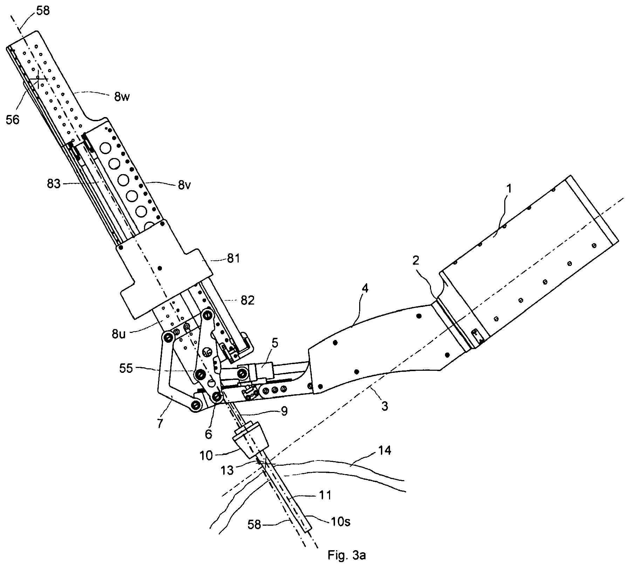

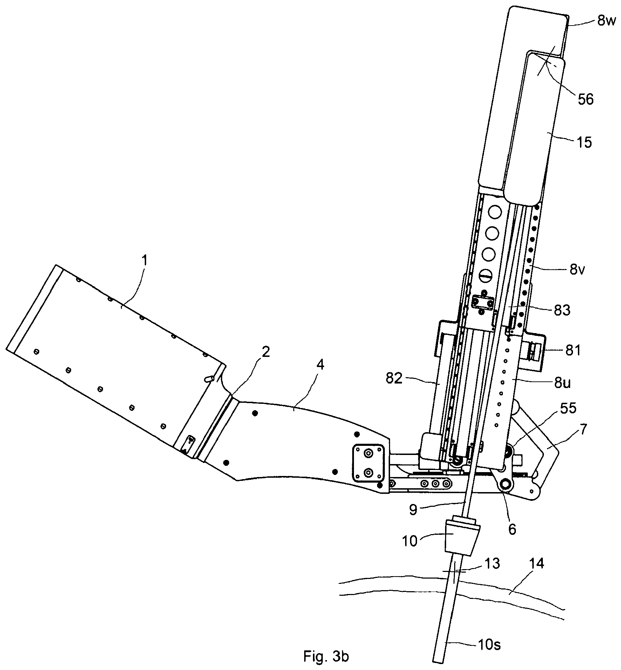

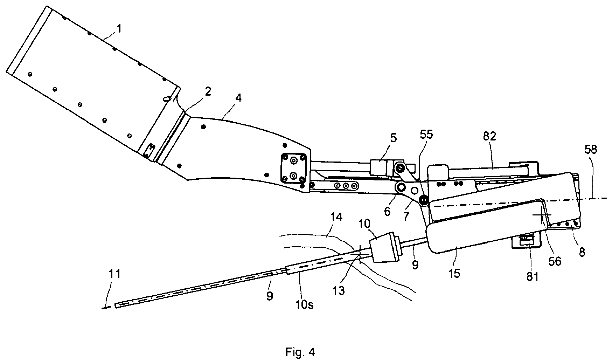

Figur 3a, Figur 3b und Figur 4 zeigen einen erfindungsgemäßen Manipulatorarm zur aktiven Positionierung eines chirurgischen Instruments 9 ohne mechanische Kopplung zwischen der Führungseinrichtung 10 zur Durchführung eines chirurgischen Instruments und der konstruktiven Vorrichtung 4 zur Realisierung der zweiten Drehachse. Gemäß dieser Ausführungsform werden die mittels Antriebseinheiten 1 und 5 erzeugten Kippbewegungen um die Drehachsen 3 und 6 nicht mechanisch auf den Pivotalpunkt 13 übertragen. Die Durchführung 10 fungiert in dieser Ausführungsform als Loslager innerhalb der Bauchdecke 14 wie es auch bei der manuellen Laparoskopie mit handgeführten Instrumenten der Fall ist. In dieser Ausführungsform resultiert die Orientierung der Instrumentenachse zwischen dem Drehpunkt 56 der Instrumentenantriebseinheit 15 und dem Drehpunkt der Führungseinrichtung 10 in der Bauchdecke 14. Der Pivotpunkt 13 in oder an der Bauchdecke 14 stellt sich aus der resultierenden Kraft zwischen von außen eingeprägten Moment und Rückstell- bzw. Haltemoment der Bauchdecke ein. Dies ist für das Gewebe der Bauchdecke, insbesondere bei der Verwendung von mehr als einem Instrumentes 9 in jeweils einer eigenen Führungseinrichtung 10, schonender, da keine direkte mechanisch fix gekoppelte Krafteinwirkung, durch das Koppelelement 12, auf die Führungseinrichtung 10 und damit auf die Bauchdecke 14 auftritt.

Der Teleskopausleger 8 dient zur Verschiebung des Instrumentes 9 durch die Führungseinrichtung 10 entlang der Instrumentenachse. Die Schubbewegung erfolgt durch das Verschieben von mindestens 2, vorzugsweise 3 Teleskopelementen 8u, 8v, 8w zueinander durch einen Stellantrieb 81 und Stellelementen 82, 83, vorzugsweise als Zahnriemen ausgeführt. Das Instrument 9 ist mittels der Instrumentenantriebseinheit 15 in dem Instrumentendrehpunkt 56 schwenkbar am äußersten Teleskopelement 8w gehaltert.

Die resultierende Instrumentenachse 11 des Instruments 9 ist aufgrund des Krafteinleitungspunktes 55 der Schubeinrichtung 5 am Teleskopausleger 8 nicht identisch mit der Teleskoplängsachse 58. Durch die schwenkbare Anordnung der Instrumentenantriebseinheit 15 am äußersten Teleskopausleger 8w und die damit mögliche Schwenk- bzw. Ausgleichsbewegung um den Instrumentendrehpunkt 56 müssen weder der Krafteinleitungspunkt 55 noch der Drehpunkt 6 des Koppelelementes 7 auf der Instrumentenlängsachse 11 liegen. Insbesondere ermöglicht die schwenkbare Anordnung der Instrumentenantriebseinheit 15 um den Instrumentendrehpunkt 56, dass die Instrumentenlängsachse 11 und die Teleskoplängsachse 58 zueinander variabel sind, wobei der Krafteinleitungspunkt 55 und der Instrumentendrehpunkt 56 unterschiedlich sind und sich gegenseitig beeinflussen.

Durch das Weglassen des Koppelelementes 12 ist die Führung von zwei chirurgischen Instrumenten 9 durch eine gemeinsame Durchführung 10 mittels zwei erfindungsgemäßer Manipulatorarme möglich und stellt gegenüber dem Stand der Technik eine wesentliche Verbesserung und erhöhte Flexibiltät dar.

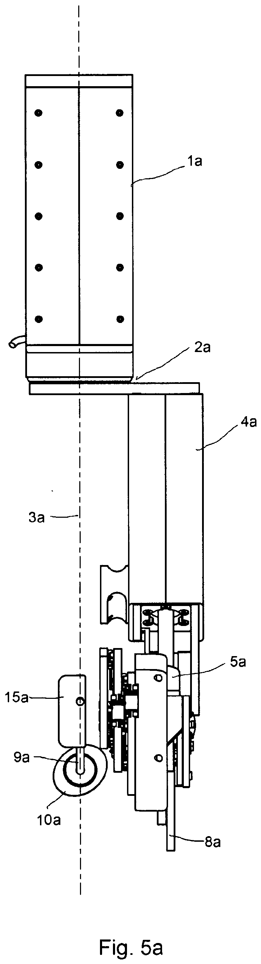

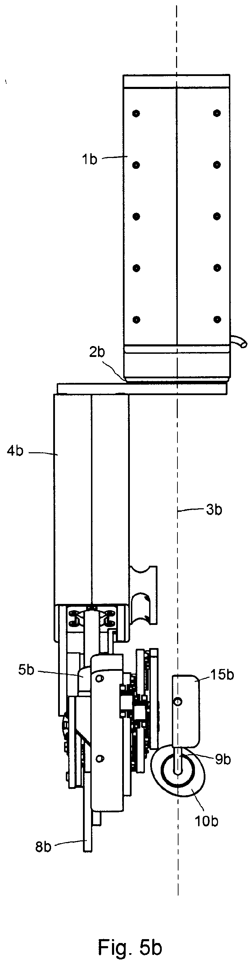

Figur 5a und 5b zeigen eine Draufsicht auf zwei verschiedene Ausführungen des erfindungsgemäßen Manipulatorarmes zur aktiven Positionierung eines chirurgischen Instrumentes. Die konstruktive Ausführung kann vorzugsweise in einer "rechtsseitigen" oder "linksseitigen" Ausführung genutzt werden. Ausgehend von der ersten Antriebseinheit 1a, 1b mit dem Drehgelenk 2a, 2b kann die zweite Antriebseinheit 4a rechts von der Drehachse 3a liegen - rechtsseitige Ausführung - bzw. kann die zweite Antriebseinheit 4b links von der Drehachse 3b liegen - linksseitige Ausführung. Die Erzeugung der zur Drehachse 3a, 3b orthogonalen Drehbewegung erfolgt analog durch die Antriebseinheit 5a, 5b. Die Bewegung des chirurgischen Instrumentes 9a, 9b entlang seiner Instrumentenlängsachse durch die Durchführung 10a, 10b erfolgt durch den Teleskopausleger 8a, 8b. Das chirurgische Instrument 9a, 9b selbst ist mittels einer Instrumentenantriebseinheit 15a, 15b mit dem Teleskopausleger 8a, 8b mechanisch verbunden.

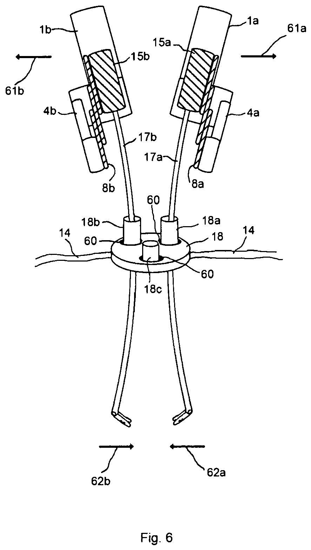

Figur 6 zeigt die Verwendung von zwei erfindungsgemäßen Manipulatorarmen zur aktiven Positionierung eines chirurgischen Instruments in den Ausführungsformen "linksseitig" und "rechtsseitig" zur gemeinsamen Verwendung mit einem Single-Port-Trokar 18 mit den Durchführungen 18a, 18b, 18c. Vorzugsweise kommen in dieser Konfiguration gekrümmte Instrumente 17a, 17b in Kombination mit einem linksseitigen Manipulatorarm 1b, 4b, 8b und einem rechtsseitigen Manipulatorarm 1a, 4a, 8a zum Einsatz mit dem Vorteil, das die chirurgischen Instrumente 17a, 17b durch einen gemeinsamen Trokar 18 - welcher den Zugang durch die Bauchdecke 14 des Patienten ermöglicht - und jeweils separaten Durchführungen 18a, 18b des gemeinsamen Trokars 18 genutzt werden können. Die separaten Durchführungen 18a, 18b und 18c des gemeinsamen Trokars 18 sind relativ zum Trokar 18 durch ein elastisches Material 60 beweglich kippbar gelagert. Durch die Möglichkeit den erfindungsgemäßen Manipulatorarm auch ohne die mechanische Kopplung 12 zwischen dem Halteelement 4 am Manipulatorarm und dem Pivotalpunkt 13 (siehe Figur 1a) zu nutzen, ist die Verwendung von nur einem Trokar 18 mit mindestens zwei Durchführungen 18a, 18b möglich. Die Verwendung eines erfindungsgemäßen linksseitigen Manipulatorarmes 1b, 4b, 8b und eines erfindungsgemäßen rechtsseitigen Manipulatorarmes 1a, 4a, 8a kann die Kollisionsgefahr zwischen den Manipulatorarmen aufgrund der pivotalen Kippbewegungen minimiert werden.

Aufgrund der vorzugsweisen Verwendung von gekrümmten Instrumenten 17a und 17b in einem Single-Port-Trokar 18 führt eine Relativbewegung 62a, 62b der beiden Instrumente aufeinander zu, z.B. um im Operationsfeld Gewebe mittels einer Naht zusammenzufügen, zu einer Relativbewegung 61a, 61b der beiden außerhalb des Patienten liegenden Manipulatorarme voneinander weg. Damit kann keine Kollision zwischen den Maipulatorarmen stattfinden.
Aus dem Stand der Technik ist die Verwendung von gekreuzten Instrumenten bei der Single-Port-Operationstechnik bekannt. Im Gegensatz dazu weist die hier vorliegende Ausführung den Vorteil der Prinzip bedingten Kollisionsvermeidung beim Zusammenführen bzw. Aufeinanderzubewegen der Instrumentenspitzen im Körper des Patienten auf.

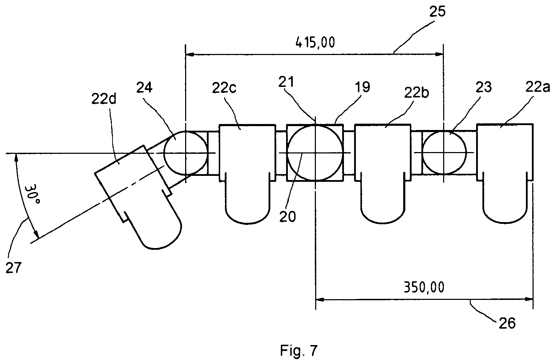

Figur 7 zeigt die konstruktive Ausführung eines flexiblen Trägersystems bzw. Halterungsträgersystem 19-26 für vorzugsweise bis zu 4 Vorpositioniereinrichtungen und Manipulatorarmen. Das flexible Trägersystem kann über eine Koppelstelle 19 an ein übergeordnetes Tragesystem derart gehaltert werden, das das flexible Trägersystem um die Drehachse 20 um mindestens ±90° in einer optimalen Position eingestellt werden kann. Das flexible Trägersystem besteht aus vorzugsweise 4 Koppelstellen 22a..d für die Adaption von bis zu vier Vorpositioniereinrichtungen. Die äußeren Koppelstellen 22a, 22d sind durch die Gelenke 23, 24 so mit den Koppelstellen 22b, 22c verbunden, das diese um bis zu 30° gegenüber der Achse 20 verkippt werden können. Die gesamte konstruktive Ausführung ist auf minimalen Bauraum 25, 26 von in etwa 415 mm bzw. 350 mm als beispielhafte Ausführungsform optimiert und kann vorzugsweise so ausgeführt werden, dass beispielsweise die Breite des flexiblen Trägersystems maximal 700mm betragen kann.

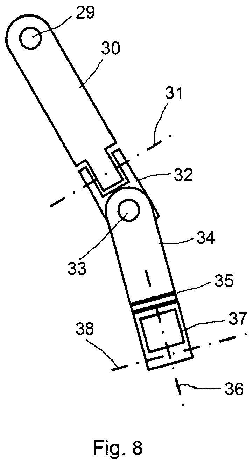

Figur 8 zeigt eine erfindungsgemäße Vorpositioniereinrichtung 29..38 zur Adaption an ein flexibles Trägersystem (Figur 7) und zur Aufnahme eines erfindungsgemäßen Manipulatorarmes (Figur 1..4). Die Vorpositioniereinrichtung wird mittels eines Koppelgelenkes 29 an eine Koppelstelle (z.B. 22d) des flexiblen Trägersystems angebracht und ermöglicht die Verdrehung eines ersten Vorpositionierelementes 30 um vorzugsweise ±90° gegenüber dem flexiblen Trägersystem, bzw. der Koppelstelle (z.B. 22d). Ein zweites Vorpositionierelement 32 ist über ein weiteres Gelenk 31 gegenüber dem ersten Vorpositionierelement 30 um weitere ±90° drehbar angeordnet. Die Drehachsen der Koppelstelle 29 und des Gelenkes 31 sind vorzugsweise orthogonal zueinander angeordnet. Das zweite Vorpositionierelement 32 ist über ein weiteres Gelenk 33 mit einem dritten Vorpositionierelement 34 derart verbunden, dass das dritte Vorpositionierelement 34 gegenüber dem zweiten Vorpositionierelement 32 um ±90° drehbar gelagert ist. Das dritte Vorpositionierelement 34 ist mit einem vierten Vorpositionierelement 37 über ein Drehgelenk 35 verbunden. Die Drehachse 36 liegt dabei vorzugsweise jeweils orthogonal zur Drehachse des Gelenkes 31 und 33 und ermöglicht Drehbewegungen um ±90°. Das vierte Vorpositionierelement 37 besitzt eine Koppelstelle, welche eine Drehbewegung um die Drehachse 38, orthogonal zur Drehachse 36 ermöglicht. An der Drehachse 38 erfolgt die Ankopplung des erfindungsgemäßen Manipulatorarmes, wie in den Figuren 1, 2, 3, 4, 5a und 5b gezeigt..

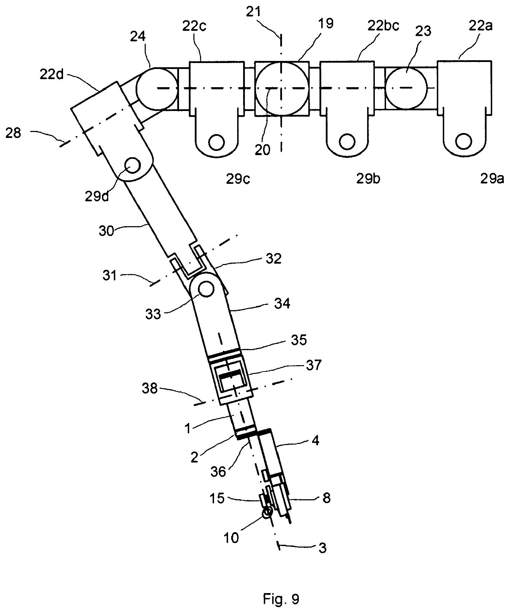

Figur 9 zeigt eine vorzugsweise Ausführungsform für die Verbindung des erfindungsgemäßen flexiblen Halterungs- und Trägersystems 19-26 mit einer erfindungsgemäßen Vorpositioniereinrichtung 29-38 mit beispielhaft einem daran angekoppelten erfindungsgemäßen Manipulatorarm 1, 2, 3, 4, 8, 10, 15. Die Antriebseinheit 1 des Manipulatorarmes ist am vierten Vorpositionierelement 37 der Vorpositioniereinrichtung in der Drehachse 38 verbunden. Die konstruktive Ausführung ist derart gestaltet, das an der Drehachse 38 der Vorpositioniereinrichtung wahlweise eine linksseitige oder rechtsseitige Ausführung des erfindungsgemäßen Manipulatorarmes angeschlossen werden kann.

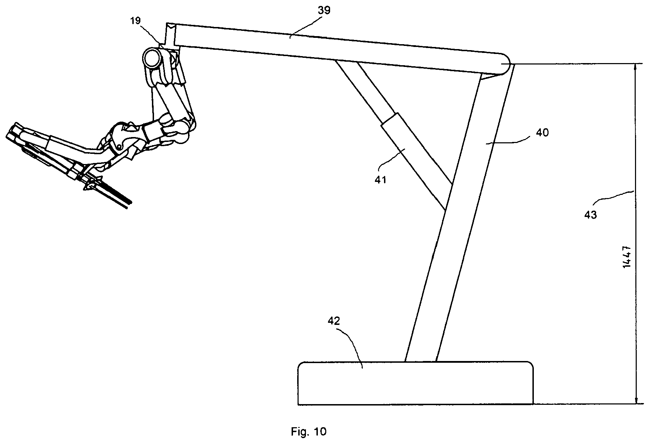

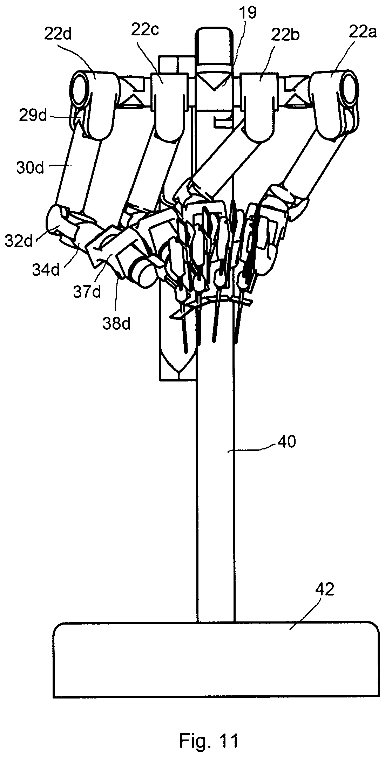

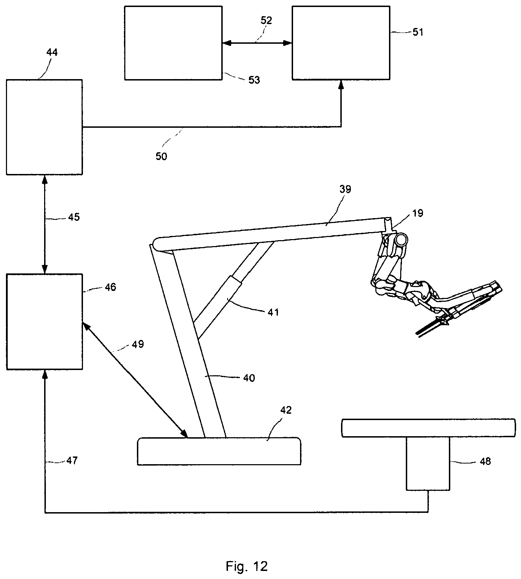

Figur 10 und Figur 11 zeigt eine konstruktive Ausführungsform des erfindungsgemäßen chirurgischen Robotersystems und insbesondere des übergeordneten Tragesystems 39 - 43 an welcher das erfindungsgemäße flexibel einstellbare Trägersystem 22a - 22d mittels der Koppelstelle bzw. Koppelträgerverbindung 19 angekoppelt ist. Das übergeordnete Tragesystem ermöglicht die optimale Vorpositionierung des flexiblen Trägersystems 22a - 22d durch eine horizontale Ausrichtung des vorzugsweise fahrbar ausgeführten Basisträgers bzw. Festlagers 42 zum OP-Tisch 48 (siehe Figur 12) und eine vertikale Ausrichtung durch die Einstellung eines optimalen Winkels zwischen der Baugruppe 39 und 40 durch das Einstellelement 41. Am erfindungsgemäßen flexiblen Trägersystem ist über die Koppelstelle 29d die erfindungsgemäße Vorpositioniereinrichtung 29d..38d befestigt und nimmt die erfindungsgemäßen Manipulatorarme an der Koppelstelle 38d auf. Die gesamte konstruktive Ausführung zeichnet sich gegenüber dem Stand der Technik dadurch aus, dass die robotischen Komponenten sämtlichst im Manipulatorarm konzentriert sind und daher die gesamte konstruktive Ausführung verglichen mit dem Stand der Technik deutlich weniger Bauraum beansprucht und insbesondere lediglich eine Höhe 43 von beispielsweise 1447 mm aufweist.

Figur 12 zeigt eine schematische Gesamtansicht der Verwendung des übergeordneten Tragesystems 39..42 in einem chirurgischen Robotersystem für den Einsatz in der minimal-invasiven Chirurgie, wie z.B. der Laparoskopie. Von einer Bedieneinheit 44 ausgehend kann der Anwender Steuerbefehle für die Aktorik des erfindungsgemäßen Manipulatorarmes über eine geeignete Datenverbindung 45 an eine Steuereinheit 46 übermitteln. Diese ist über eine weitere Datenleitung 49 mit dem übergeordneten Tragesystem 39..42 verbunden und, ausgestattet mit einem Tragarm bzw. Hauptträgereinrichtung 39, 40, kann ein über die Koppelstelle 19 angeschlossenes flexibles Trägersystem entsprechend der Patientenposition auf dem OP-Tisch 48 über eine die Koppelstelle 19 so vorpositioniert werden, dass das flexibles Trägersystem in Verbindung mit den Vorpositioniereinrichtungen eine optimale Positionierung der Manipulatorarme ermöglicht.

Bei Ausstattung eines erfindungsgemäßen Manipulatorarmes mit z.B. einer Endoskop-Kamera können die Bildsignale über geeignete Datenverbindungen 49, 45, 50 einer Verarbeitungseinheit 51 zugeführt werden, welche die Bilddaten zur Darstellung aufbereitet und über eine weitere Datenstrecke 52 einer Visualisierungseinheit 53 zuführt. Die Visualisierungseinheit 53 kann sowohl 2D- als auch 3D-Bilddaten darstellen, beispielsweise getrennt, jedoch auch kombiniert in einem einzigen Bild bzw. einer einzigen Bildabfolge. Die Steuerung, welche Bilddaten wie dargestellt werden sollen, erfolgt durch die Steuereinheit 44 je nach Wunsch des Operators bzw. Chirurgen. Die dazu von der Steuereinheit 44 erzeugten Steuerbefehle werden mittels der Datenstrecke 50 zur Verarbeitungseinheit 51 übertragen.

Gemäß einer weiteren Ausführungsform ist die erfindungsgemäße Vorrichtung derart ausgebildet, dass an der Teleskopeinrichtung eine Instrumentenführungseinreichtung angebracht ist, durch welche das chirurgische Instrument in einer Ebene quer zur Längserstreckung geführt wird, wobei die Instrumentenführungseinrichtung insbesondere eine Führungsöffnung zur variablen Positionierung des chirurgischen Instruments aufweist. Die zusätzliche Instrumentenführungseinrichtung ist an der Teleskopeinrichtung angebracht, in welcher sich der Schaft des chirurgischen Instrumentes und/oder Endoskops hindurch erstreckt. Diese zusätzliche Instrumentenführungseinrichtung ist starr mit der Teleskopeinrichtung verbunden. Durch diese zusätzliche Instrumentenführungseinrichtung wird das chirurgische Instrument und/oder Endoskop bei Drehung des Manipulatorarmes um die erste Drehachse zwangsgeführt. Durch die konstruktive Ausführung der zusätzlichen Instrumentenführungseinrichtung erfolgt die Zwangsführung nur für Bewegungen des Manipulatorarmes um die erste Drehachse. Bei Drehungen des Manipulatorarmes um die zweite Drehachse ermöglicht die zusätzliche Instrumentenführungseinrichtung eine freie Bewegung des chirurgischen Instrumentes und/oder Endoskops derart, das sich eine resultierende Instrumentenachse, aus der Drehung der Instrumentenantriebseinheit an der Teleskopeinrichtung und der Lage einer ersten Führungseinrichtung (Trokar), durch welchen sich das chirurgische Instrument und/oder Teleskop hindurch erstreckt, einstellt.

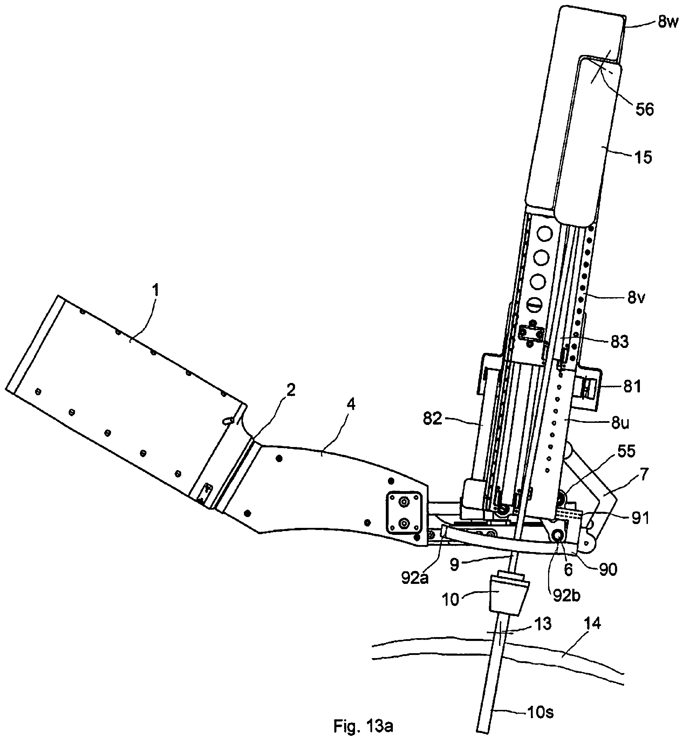

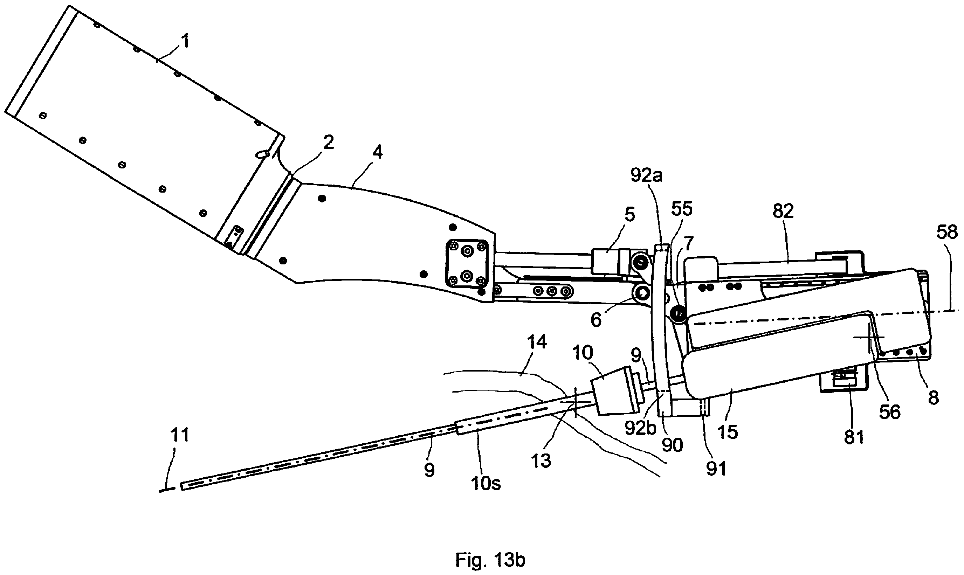

Die Figuren 13a, 13b, 14a, 14b, 15a, 15b zeigen einen erfindungsgemäßen Manipulatorarm zur aktiven Positionierung eines chirurgischen Instruments 9 ohne mechanische Kopplung zwischen der Führungseinrichtung 10 zur Durchführung eines chirurgischen Instruments 9 und der konstruktiven Vorrichtung 4 zur Realisierung der zweiten Drehachse. Die Figuren 13 a und 13 b zeigen eine Ausführungsform der Erfindung, welche im wesentlichen derjenigen der Figuren 3 a und 3 b mit einer Instrumentenführungseinrichtung 90 entspricht.

Am Teleskopausleger 8 ist die Instrumentenführungseinrichtung 90 mittels einer wiederlösbaren Befestigungsvorrichtung 91 insbesondere in Form einer Schraube derart angebracht, das bei Drehung des Manipulatorarmes um eine erste Drehachse (Drehung des Drehgelenkes 2) das chirurgische Instrument 9 durch bzw. innerhalb der Instrumentenführungseinrichtung 90 zwangsweise geführt wird. Die Instrumentenführungseinrichtung 90 ist konstruktiv so ausgeführt, dass das chirurgische Instrument 9 bei Verkippung des Manipulatorarmes um eine zweite Drehachse 2 (Drehung um den Drehpunkt 6) sich innerhalb der Instrumentenführungseinrichtung 90 in einer Längsöffnung 92 zwischen den Begrenzungen 92a und 92b der Längsöffnung 92 frei in einer Achse verschieben kann, so dass sich eine resultierende Ausrichtung der Längsachse des chirurgischen Instrumentes 9 ohne Zwangsführung aus dem Instrumentendrehpunkt 56 und der Führungseinrichtung 10 ergibt.

Diese Lösung hat den Vorteil, das bei Drehung eines chirurgischen Instrumentes 9 um eine erste Drehachse (Drehung um Drehgelenk 2) das chirurgischen Instrument 9 zwangsgeführt wird und die zur Zwangsführung verwendete Instrumentenführungseinrichtung 90 auf das chirurgische Instrument 9 einwirkende Kräfte in Richtung der Drehachse 6 aufgenommen werden, ohne die Instrumentendurchführung 10 mit dem Manipulatorarm mechanisch verkoppeln bzw. verbinden zu müssen.

Die Figur 13b verdeutlicht die freie Schwenkbarkeit des Instrumentes 9 in der Instrumentenführungseinrichtung 90 zwischen den Begrenzungen 92a und 92b der Führungsöffnung.

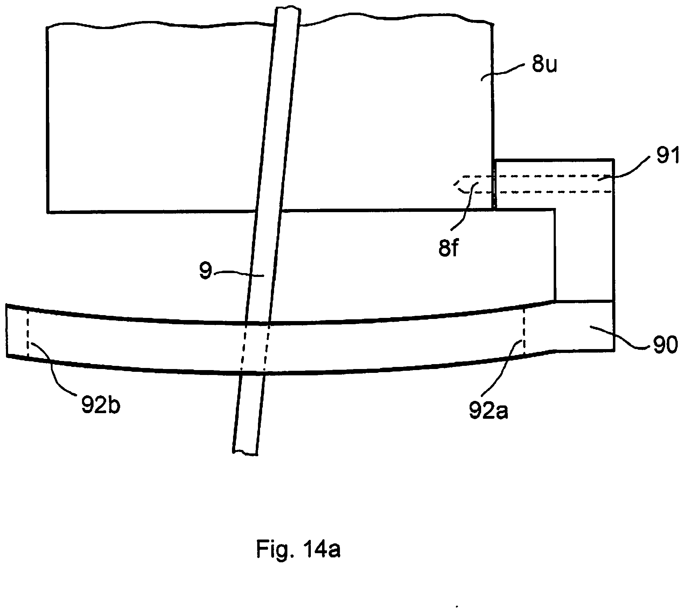

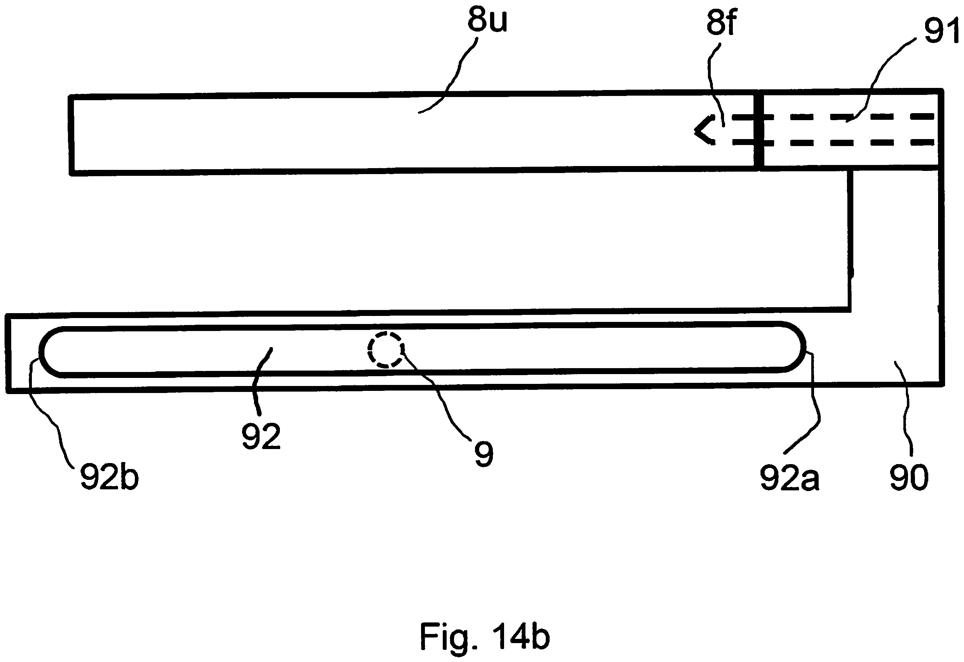

Die Figuren 14a, 14b zeigen schematisch als Ausschnitt die Ausführungsform von Figur 13 a und 13 b mit der Instrumentenführungseinrichtung 90, welche an den Teleskopausleger 8u mittels einer nicht gezeigten Schraube in der Befestigungsvorrichtung 8f , 91 befestigt ist, vorzugsweise als wiederlösbare Schraub- oder Steckverbindung ausgeführt. Des weiteren wird die längliche Führungsöffnung 92 mit ihren seitlichen Begrenzungen 92a und 92b dargestellt, zwischen denen das chirurgische Instrument 9 verschoben werden kann.

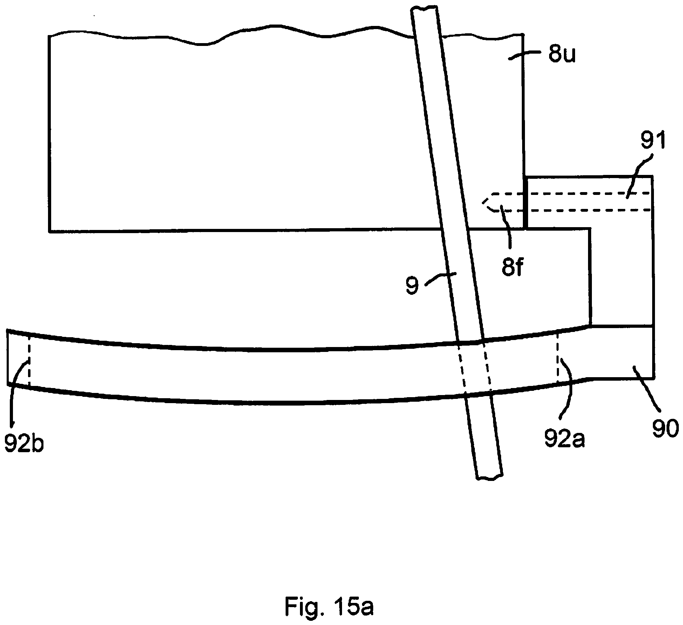

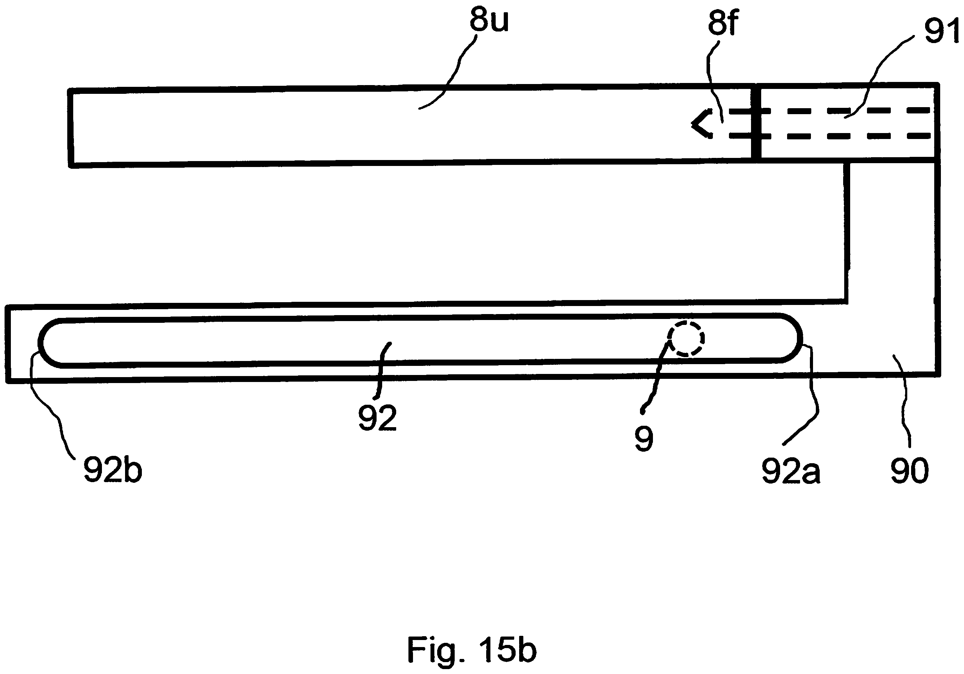

Figuren 15a, 15b zeigen schematisch ebenfalls die Ankopplung der Instrumentenführungseinrichtung 90 an den Teleskopausleger 8u für eine weitere Orientierung des chirurgischen Instrumentes 9 gegenüber der Längsachse des Teleskopes 8u, wodurch ersichtlich ist, dass das das chirurgischen Instrument 9 innerhalb der Instrumentenführungseinrichtung 90 in seiner Position bewegbar ist.

Die Ausführungsform der Erfindung gemäß der Figuren 13 bis 15 mit der Instrumentenführungseinrichtung 90 hat insbesondere den Vorteil, dass keine Abhängigkeit von der mechanischen Belastungsgrenze des Trokars bzw. der Instrumentendurchführung 10 besteht.

Des Weiteren ermöglicht es die Instrumentenführungseinreichtung 90, dass sich durch die nach wie vor vorhandene Entkoppelung der Längsachse des chirurgischen Instruments 9 von der Längsachse des Teleskopauslegers 8 ein resultierender Pivotpunkt 13 an der Stelle einstellt, an welcher die biomechanische Belastungen für die Bauchdecke durch das Wegkippen der Instrumentenführung 10 minimal sind.

Die vorliegende Erfindung bezieht sich somit einerseits auf eine Halterungs- und Positioniervorrichtung für ein chirurgisches Instrument und/oder ein Endoskop, wobei eine oder mehrere derartige Halterungs- und Positioniervorrichtungen gemäß der Erfindung an einem chirurgischen Robotersystem jeweils über Koppelstellen angebracht sind, wobei diese Koppelstellen wiederum jeweils miteinander verbunden sind, sodass der benötigte Bauraum des chirurgischen Robotersystems in vorteilhafter Weise nur sehr gering ist. Die besonders kompakte Bauweise ergibt sich des Weiteren durch die besonders leichte und kompakte Ausführbarkeit der erfindungsgemäßten Halterungs- und Positioniervorrichtung, wobei diese des Weiteren auch an einem bestehenden Robotersystem nachgerüstet werden kann.

In einer bevorzugten Ausführungsform ist die Führungseinrichtung zur Durchführung eines chirurgischen Instruments über ein Koppelelement mit der konstruktiven Vorrichtung zur Erzeugung der zweiten Drehachse starr verbunden. Die Drehbewegung der Drehachse 1 führt damit zu einer Zwangsbewegung der Führungseinrichtung zur Durchführung eines chirurgischen Instruments um den invarianten Punkt in einer Richtung x.

In einer weiteren bevorzugten Ausführungsform ist die Führungseinrichtung zur Durchführung eines chirurgischen Instruments nicht mit der konstruktiven Vorrichtung zur Erzeugung der zweiten Drehachse konstruktiv starr verbunden. Damit fungiert die Führungseinrichtung zur Durchführung eines chirurgischen Instruments als Loslager in der Bauchdecke wie in der manuellen Laparoskopie üblich.

In einer ferner bevorzugten Ausführungsform ist an das chirurgische Instrument über eine Instrumentenantriebseinheit an die Teleskopvorrichtung gekoppelt, welche einen Rotationsaktuator umfasst, durch den der Schaft des chirurgischen Instruments gegenüber der Ausgangslage um die z-Richtung drehbar variiert wird. Bevorzugt weist die Instrumentenantriebseinheit drei Instrumentenaktuatoren auf, durch welche die am distalen Ende angebrachte Wirkeinheit des chirurgischen Instruments in drei weiteren Freiheitsgraden variierbar ist. Insbesondere bevorzugt ist die Instrumentenantriebseinheit über eine Halteeinrichtung drehbar an dem proximalen Ende des Teleskopsystems angeordnet.

## Patentansprüche

1. Halterungs- und Positioniervorrichtung eines Instruments für die minimal-invasive Chirurgie, zur Verwendung innerhalb eines chirurgischen Robotersystems, umfassend
- eine Instrumentenantriebseinheit (15),
- eine Teleskopeinrichtung (8), an welcher ein chirurgisches Instrument (9; 17a, 17b) mittels der Instrumentenantriebseinheit (15) befestigt ist, wobei das chirurgische Instrument (9; 17a, 17b) durch die Teleskopeinrichtung (8) entlang seiner Instrumentenlängsachse (11) durch eine Führungseinrichtung (10, 10s) in den Körper translatorisch bewegbar ist, ,
- eine Antriebseinheit (1), die eine erste Drehachse (3) aufweist, um welche ein Halteelement (4) drehbar angeordnet ist, wobei sich die erste Drehachse (3) mit der Instrumentenlängsachse (11) des chirurgischen Instruments (9; 17a, 17b) in einem Pivotalpunkt (13) stets schneidet,
- **dadurch gekennzeichnet, dass** an dem Halteelement (4) ein Schubantrieb (5) angebracht ist, und die Teleskopeinrichtung (8) an einer als Koppelgelenk ausgestalteten Koppelführung (7) angeordnet ist, wobei der Schubantrieb (5) Kraft auf einen als Drehpunkt an der Koppelführung (7) ausgebildeten Schubantriebsaufnahmepunkt (55) einleitend ausgebildet ist,
- wobei die Instrumentenantriebseinheit (15) an der Teleskopeinrichtung (8) mittels eines Instrumentendrehpunkts (56) drehbar gelagert ist und die Koppelführung (7) einen Kopplungsdrehpunkt (6) aufweist, welcher mit dem Halteelement (4) fest verbunden ist und eine zweite Drehachse definiert, welche senkrecht zur ersten Drehachse (3) und von dieser beabstandet angeordnet ist, so dass mittels Krafteinleitung über den Schubantrieb (5) auf die Koppelführung (7) eine Rotation der Koppelführung (7) um die zweite Drehachse realisierbar ist, wodurch die Instrumentenlängsachse (11) des chirurgischen Instruments (9; 17a, 17b) gegenüber einer Teleskoplängsachse (58) der Teleskopeinrichtung (8) in Abhängigkeit des Schubantriebs (5) variabel einstellbar ist und die Instrumentenantriebseinheit (15) um eine zur ersten Drehachse (3) orthogonale Achse um den Pivotalpunkt (13) drehbar ist.

2. Halterungs- und Positioniervorrichtung nach Anspruch 1, **dadurch gekennzeichnet, dass** die Teleskopeinrichtung (8) mehrere Teleskopelemente (8u, 8v, 8w) aufweist, wobei der Instrumentendrehpunkt (56) an dem Teleskopelement (8w) angeordnet ist, welches die größte teleskopartige Verstellmöglichkeit aufweist.

3. Halterungs- und Positioniervorrichtung nach einem der vorhergehenden Ansprüche 1 oder 2, **dadurch gekennzeichnet, dass** die Führungseinrichtung (10, 10s) zumindest eine Instrumentenführung (10s) aufweist, in welcher sich der Schaft des chirurgischen Instruments (9; 17a, 17b) hindurch erstreckt.

4. Halterungs- und Positioniervorrichtung nach einem der vorhergehenden Ansprüche 1 bis 3, **dadurch gekennzeichnet, dass** die Instrumentenantriebseinheit (15) das chirurgische Instrument (9; 17a, 17b) in mehreren Freiheitsgraden bewegt, wobei die Ansteuerung der Instrumentenantriebseinheit (15) mittels Steuer- und Versorgungsleitungen, welche durch das Haltelement (4) und den Schubantrieb (5) hindurch geführt werden, über eine Steuereinheit (44) durch den Operateur erfolgt.

5. Halterungs- und Positioniervorrichtung nach einem der vorhergehenden Ansprüche 1 bis 4, **dadurch gekennzeichnet, dass** die erste Drehachse (3) dadurch gebildet wird, dass eine Antriebseinheit (1) vorgesehen ist, die an einem Roboterarm anbringbar ist, wobei ein Drehgelenk (2) zwischen der Antriebseinheit (1) und dem Halteelement (4) vorgesehen ist.

6. Halterungs- und Positioniervorrichtung nach einem der vorhergehenden Ansprüche 1 bis 5, **dadurch gekennzeichnet, dass** ein Koppelelement (12) am Halteelement (4) angebracht ist, welches am distalen Ende am Pivotalpunkt (13) mit der Instrumentenführung (10s) drehbar verbunden ist.

7. Halterungs- und Positioniervorrichtung nach einem der vorhergehenden Ansprüche 1 bis 6, **dadurch gekennzeichnet, dass** mehrere chirurgische Instrumente (17a, 17b) durch einen einzigen Trokar (18) hindurch in das Körperinnere geführt werden, wobei für jedes chirurgische Instrument (17a, 17b) eine separate Instrumentenantriebseinheit (15a, 15b) vorgesehen ist, und wobei insbesondere die chirurgischen Instrumente (17a, 17b) in Längsrichtung bogenförmig ausgebildet sind.

8. Halterungs- und Positioniervorrichtung nach einem der vorhergehenden Ansprüche 1 bis 7, **dadurch gekennzeichnet, dass** das Halteelement (4) und/oder die Antriebseinheit (1) mittels einer Vorpositioniereinrichtung in seiner Ausgangslage anpassbar ist, wobei die Vorpositioniereinrichtung ein oder mehrere Vorpositionierelemente (30, 32, 34, 37) aufweist, welche jeweils über zumindest eine Drehachse in ihrer Position voreinstellbar sind, wobei insbesondere vier Vorpositionierelemente (30, 32, 34, 37) mit zueinander in Reihe variablen Positionen voreinstellbar sind.

9. Halterungs- und Positioniervorrichtung nach einem der vorhergehenden Ansprüche 1 bis 8, **dadurch gekennzeichnet, dass** an der Teleskopeinrichtung (8) eine Instrumentenführungseinreichtung (90) angebracht ist, durch welche das chirurgische Instrument (9) in einer Ebene quer zur Längserstreckung geführt wird, wobei die Instrumentenführungseinrichtung (90) insbesondere eine Führungsöffnung (92) zur variablen Positionierung des chirurgischen Instruments (9) aufweist.

10. Chirurgisches Robotersystem mit mehreren Halterungs- und Positioniervorrichtungen gemäß einem der Ansprüche 1 bis 9, wobei zumindest zwei Halterungs- und Positioniervorrichtungen an einem im wesentlichen quer zu den Halterungs- und Positioniervorrichtungen verlaufenden Halterungsträgersystem (19, 20, 21, 22, 23, 24) angebracht sind, wobei das Halterungsträgersystem (19, 20, 21, 22, 23, 24) aus jeweils einer Koppelstelle (22a-d) für jede Halterungs- und Positioniervorrichtung aufgebaut ist, und wobei die Koppelstellen (22a-d) jeweils fest oder über Gelenke (23, 24) miteinander verbunden sind.

11. Chirurgisches Robotersystem nach Anspruch 10, **dadurch gekennzeichnet, dass** das Halterungsträgersystem (19, 20, 21, 22, 23, 24) mittels einer Koppelträgerverbindung (19) mit einer im wesentlichen vertikal verlaufenden Hauptträgereinrichtung (39, 40) zur Abstützung gegenüber einem Festlager (42) verbunden ist, welches beweglich angeordnet oder gegenüber einem feststehenden oder verfahrbaren Operationstisch (48) vorgegeben ist.

12. Chirurgisches Robotersystem nach Anspruch 10 oder 11, **dadurch gekennzeichnet, dass** eine zentrale Steuereinheit (46) vorgesehen ist, welche mit jeder der Halterungs- und Positioniervorrichtungen mit den entsprechenden chirurgischen Instrumenten (9; 17a, 17b) und/oder Endoskopen (9; 17a, 17b) in Verbindung steht und mit einer Bedieneinheit (44) zur Eingabe von Befehlen in Form von Steuerdaten eines Operateurs gekoppelt ist, welche mittels einer Visualisierungseinheit (53) Bilddaten von einem oder mehrerer Endoskopen (9; 17a, 17b) darstellt.

13. Chirurgisches Robotersystem nach einem der Ansprüche 10 bis 12, **dadurch gekennzeichnet, dass** die Steuereinheit (46) und die Bedieneinheit (44) mit einem verfahrbaren Operationstisch (48) gekoppelt sind, wobei sowohl die Bilddaten als auch die Steuerdaten in Abhängigkeit der vorgegebenen Positionen der Halterungs- und Positioniervorrichtung sowie des Operationstisches (48) verarbeitet werden.

## Claims

1. Holding and positioning device of an instrument for minimally invasive surgery, for use within a surgical robot system, comprising
- an instrument drive unit (15),
- a telescopic device (8) to which a surgical instrument (9; 17a, 17b) is secured by means of the instrument drive unit (15), wherein the surgical instrument (9; 17a, 17b) can be moved into the body translationally by the telescopic device (8) along its instrument longitudinal axis (11) through a guide device (10, 10s),
- a drive unit (1) which has a first axis of rotation (3) about which a holding element (4) is arranged rotatable, wherein the first axis of rotation (3) always intersects with the instrument longitudinal axis (11) of the surgical instrument (9; 17a, 17b) in a pivot point (13),
- **characterized in that** a linear actuator (5) is attached to the holding element (4), and the telescopic device (8) is arranged on a coupling guide (7) formed as a coupling joint, wherein the linear actuator (5) is formed to transmit force to a linear actuator position point (55) formed as a pivotal point on the coupling guide (7),
- wherein the instrument drive unit (15) is mounted rotatable on the telescopic device (8) by means of an instrument pivotal point (56) and the coupling guide (7) has a coupling pivotal point (6) which is rigidly connected to the holding element (4) and defines a second axis of rotation which is arranged perpendicular to the first axis of rotation (3) and at a distance from same, with the result that by means of force transmission via the linear actuator (5) to the coupling guide (7) a rotation of the coupling guide (7) about the second axis of rotation can be realized, whereby the instrument longitudinal axis (11) of the surgical instrument (9; 17a, 17b) is variably adjustable relative to a telescopic longitudinal axis (58) of the telescopic device (8) as a function of the linear actuator (5), and the instrument drive unit (15) is rotatable about the pivot point (13) about an axis orthogonal to the first axis of rotation (3).

2. Holding and positioning device according to claim 1, **characterized in that** the telescopic device (8) has several telescopic elements (8u, 8v, 8w), wherein the instrument pivotal point (56) is arranged on the telescopic element (8w) which has the largest telescopic adjustability.

3. Holding and positioning device according to one of the preceding claims 1 or 2, **characterized in that** the guide device (10, 10s) has at least one instrument guide (10s) through which the shaft of the surgical instrument (9; 17a, 17b) extends.

4. Holding and positioning device according to one of the preceding claims 1 to 3, **characterized in that** the instrument drive unit (15) moves the surgical instrument (9; 17a, 17b) in several degrees of freedom, wherein the instrument drive unit (15) is controlled by the surgeon via a control unit (44) by means of control and supply lines which are guided through the holding element (4) and the linear actuator (5).

5. Holding and positioning device according to one of the preceding claims 1 to 4, **characterized in that** the first axis of rotation (3) is formed **in that** a drive unit (1) is provided which can be attached to a robot arm, wherein a pivot joint (2) is provided between the drive unit (1) and the holding element (4).

6. Holding and positioning device according to one of the preceding claims 1 to 5, **characterized in that** a coupling element (12), which is rotatably connected to the instrument guide (10s) at the pivot point (13) at the distal end, is attached to the holding element (4).

7. Holding and positioning device according to one of the preceding claims 1 to 6, **characterized in that** several surgical instruments (17a, 17b) are guided into the inside of the body through a single trocar (18), wherein a separate instrument drive unit (15a, 15b) is provided for each surgical instrument (17a, 17b), and wherein in particular the surgical instruments (17a, 17b) are formed curved in the longitudinal direction.

8. Holding and positioning device according to one of the preceding claims 1 to 7, **characterized in that** the holding element (4) and/or the drive unit (1) can be adapted in its starting position by means of a pre-positioning device, wherein the pre-positioning device has one or more pre-positioning elements (30, 32, 34, 37) which in each case can be pre-set in their position via at least one axis of rotation, wherein in particular four pre-positioning elements (30, 32, 34, 37) can be pre-set with positions variable relative to one another in series.

9. Holding and positioning device according to one of the preceding claims 1 to 8, **characterized in that** there is attached to the telescopic device (8) an instrument guide device (90) by which the surgical instrument (9) is guided in a plane transverse to the longitudinal extension, wherein the instrument guide device (90) in particular has a guide opening (92) for the variable positioning of the surgical instrument (9).

10. Surgical robot system with several holding and positioning devices according to one of claims 1 to 9, wherein at least two holding and positioning devices are attached to a holding support system (19, 20, 21, 22, 23, 24) running substantially transverse to the holding and positioning devices, wherein the holding support system (19, 20, 21, 22, 23, 24) is constructed in each case from a coupling point (22a-d) for each holding and positioning device, and wherein the coupling points (22a-d) in each case are connected to one another rigidly or via joints (23, 24).

11. Surgical robot system according to claim 10, **characterized in that** the holding support system (19, 20, 21, 22, 23, 24) is connected by means of a coupling support connection (19) to a substantially vertically running main support device (39, 40) for support in relation to a fixed bearing (42) which is arranged movable or predefined with respect to a stationary or mobile operating table (48).

12. Surgical robot system according to claim 10 or 11, **characterized in that** a central control unit (46) is provided which is connected to each of the holding and positioning devices with the corresponding surgical instruments (9; 17a, 17b) and/or endoscopes (9; 17a, 17b) and is coupled to an operating unit (44), for entering commands in the form of control data of a surgeon, which displays image data from one or more endoscopes (9; 17a, 17b) by means of a visualization unit (53).

13. Surgical robot system according to one of claims 10 to 12, **characterized in that** the control unit (46) and the operating unit (44) are coupled to a mobile operating table (48), wherein both the image data and the control data are processed according to the predefined positions of the holding and positioning device as well as of the operating table (48).

## Revendications

1. Dispositif de retenue et de positionnement d'un instrument de chirurgie minimalement invasive, destiné à être utilisé dans un système chirurgical robotisé, comprenant
- une unité d'entraînement d'instrument (15),
- un dispositif télescopique (8) auquel un instrument chirurgical (9 ; 17a, 17b) est fixé au moyen de l'unité d'entraînement d'instrument (15), dans lequel l'instrument chirurgical (9 ; 17a, 17b) peut être déplacé à l'intérieur du corps par translation par le dispositif télescopique (8) le long de son axe longitudinal d'instrument (11) à travers un dispositif de guidage (10, 10s),
- une unité d'entraînement (1) qui présente un premier axe de rotation (3) autour duquel un élément de retenue (4) est agencé avec faculté de rotation, dans lequel le premier axe de rotation (3) intersecte toujours l'axe longitudinal d'instrument (11) de l'instrument chirurgical (9 ; 17a, 17b) à un point pivot (13),
- **caractérisé en ce qu'**un actionneur linéaire (5) est rattaché à l'élément de retenue (4), et le dispositif télescopique (8) est agencé sur un guide de couplage (7) formé sous forme d'articulation de couplage, dans lequel l'actionneur linéaire (5) est formé pour transmettre une force à un point de position d'actionneur linéaire (55) formé en tant que point pivot sur le guide de couplage (7),
- dans lequel l'unité d'entraînement d'instrument (15) est montée avec faculté de rotation sur le dispositif télescopique (8) au moyen d'un point de pivotement d'instrument (56) et le guide de couplage (7) présente un point de pivotement de couplage (6) qui est connecté de manière rigide à l'élément de retenue (4) et définit un second axe de rotation agencé perpendiculairement au premier axe de rotation (3) et à une distance de celui-ci, ceci faisant qu'au moyen d'une transmission de force par l'intermédiaire de l'actionneur linéaire (5) au guide de couplage (7) une rotation du guide de couplage (7) autour du second axe de rotation peut être réalisée, moyennant quoi l'axe longitudinal d'instrument (11) de l'instrument chirurgical (9 ; 17a, 17b) est réglable de manière variable par rapport à un axe longitudinal télescopique (58) du dispositif télescopique (8) en fonction de l'actionneur linéaire (5), et l'unité d'entraînement d'instrument (15) peut tourner autour du point pivot (13) autour d'un axe orthogonal au premier axe de rotation (3).

2. Dispositif de retenue et de positionnement selon la revendication 1, **caractérisé en ce que** le dispositif télescopique (8) présente plusieurs éléments téléscopiques (8u, 8v, 8w), dans lequel le point de pivotement d'instrument (56) est agencé sur l'élément télescopique (8w) qui présente la plus grande possibilité d'ajustement télescopique.

3. Dispositif de retenue et de positionnement selon l'une des revendications précédentes 1 ou 2, **caractérisé en ce que** le dispositif de guidage (10, 10s) présente au moins un guide d'instrument (10s) au travers duquel passe l'arbre de l'instrument chirurgical (9 ; 17a, 17b).

4. Dispositif de retenue et de positionnement selon l'une des revendications précédentes 1 à 3, **caractérisé en ce que** l'unité d'entraînement d'instrument (15) déplace l'instrument chirurgical (9 ; 17a, 17b) par plusieurs degrés de liberté, dans lequel l'unité d'entraînement d'instrument (15) est commandée par le chirurgien par l'intermédiaire d'une unité de commande (44) au moyen de lignes de commande et d'alimentation électrique qui sont guidées à travers l'élément de retenue (4) et l'actionneur linéaire (5).

5. Dispositif de retenue et de positionnement selon l'une des revendications précédentes 1 à 4, **caractérisé en ce que** le premier axe de rotation (3) est formé **en ce qu'**une unité d'entraînement (1) est fournie qui peut être rattachée à un bras robotique, dans lequel une articulation pivot (2) est fournie entre l'unité d'entraînement (1) et l'élément de retenue (4).

6. Dispositif de retenue et de positionnement selon l'une des revendications précédentes 1 à 5, **caractérisé en ce qu'**un élément de couplage (12), qui est raccordé en rotation au guide d'instrument (10s) au niveau du point pivot (13) à l'extrémité distale, est rattaché à l'élément de retenue (4).

7. Dispositif de retenue et de positionnement selon l'une des revendications précédentes 1 à 6, **caractérisé en ce que** plusieurs instruments chirurgicaux (17a, 17b) sont guidés à l'intérieur du corps à travers un seul trocart (18), dans lequel une unité d'entraînement d'instrument séparée (15a, 15b) est fournie pour chaque instrument chirurgical (17a, 17b), et dans lequel en particulier les instruments chirurgicaux (17a, 17b) sont formés en forme d'arc dans le sens longitudinal.

8. Dispositif de retenue et de positionnement selon l'une des revendications précédentes 1 à 7, **caractérisé en ce que** l'élément de retenue (4) et/ou l'unité d'entraînement (1) peut être adapté en termes de sa position de départ au moyen d'un dispositif de pré-positionnement, dans lequel le dispositif de pré-positionnement présente un ou plusieurs éléments de pré-positionnement (30, 32, 34, 37) qui dans chaque cas peuvent être préréglés en termes de leur position par l'intermédiaire d'au moins un axe de rotation, dans lequel en particulier quatre éléments de pré-positionnement (30, 32, 34, 37) peuvent être préréglés avec des positions variables les unes par rapport aux autres en série.

9. Dispositif de retenue et de positionnement selon l'une des revendications précédentes 1 à 8, **caractérisé en ce qu'**au dispositif télescopique (8) est rattaché un dispositif de guidage d'instrument (90) par lequel l'instrument chirurgical (9) est guidé dans un plan transversal à l'extension longitudinale, dans lequel le dispositif de guidage d'instrument (90) en particulier comporte une ouverture de guidage (92) pour le positionnement variable de l'instrument chirurgical (9).

10. Système chirurgical robotisé doté de plusieurs dispositifs de retenue et de positionnement selon l'une des revendications 1 à 9, dans lequel au moins deux dispositifs de retenue et de positionnement sont rattachés à un système de support de retenue (19, 20, 21, 22, 23, 24) qui s'étend sensiblement transversalement aux dispositifs de retenue et de positionnement, dans lequel le système de support de retenue (19, 20, 21, 22, 23, 24) est construit dans chaque cas à partir d'un point de couplage (22a-d) pour chaque dispositif de retenue et de positionnement, et dans lequel les points de couplage (22a-d) dans chaque cas sont raccordés l'un à l'autre rigidement ou par l'intermédiaire d'articulations (23, 24).

11. Système chirurgical robotisé selon la revendication 10, **caractérisé en ce que** le système de support de retenue (19, 20, 21, 22, 23, 24) est raccordé au moyen d'une connexion de support de couplage (19) à un dispositif de support principal (39, 40) s'étendant sensiblement verticalement en vue d'un support relativement à un palier fixe (42) qui est agencé pour pouvoir se déplacer ou prédéfini par rapport à une table d'opération (48) fixe ou mobile.

12. Système chirurgical robotisé selon la revendication 10 ou 11, **caractérisé en ce qu'**est fournie une unité centrale de commande (46) qui est connectée à chacun des dispositifs de retenue et de positionnement avec les instruments chirurgicaux correspondants (9 ; 17a, 17b) et/ou endoscopes (9 ; 17a, 17b) et est couplée à une unité d'opération (44), destinée à l'entrée de commandes sous forme de données de commande fournies par un chirurgien, laquelle affiche des données d'image provenant d'un ou de plusieurs endoscopes (9 ; 17a, 17b) au moyen d'une unité de visualisation (53).

13. Système chirurgical robotisé selon l'une des revendications 10 à 12, **caractérisé en ce que** l'unité de commande (46) et l'unité d'opération (44) sont couplées à une table d'opération mobile (48), dans lequel à la fois les données d'image et les données de commande sont traitées conformément aux positions prédéfinies du dispositif de retenue et de positionnement ainsi que de la table d'opération (48).
